# EUROPEAN PATENT APPLICATION

(11) **EP 3 988 129 A1**
(43) Date of publication of application: **27.04.2022**
(21) Application number: 20207970.3
(22) Date of filing: 17.11.2020
(51) Int. Cl.: A61L 2/10, A61L 2/24, A61L 9/14, A61L 9/20

(54) **INDOOR AIR STERILIZATION AND PURIFICATION APPARATUS USING UV-C LED**

(30) Priority: 22.10.2020 KR 20200137297
(71) Applicant: Aden Hygiene, Ent., Cheongju-si, Chungcheongbuk-do 28160 (KR)
(72) Inventor: CHOI, Woo-Sung, 16507 Gyeonggi-do (KR); CHO, Meoungwhan, 16990 Gyeonggi-do (KR); LEE, Seogwoo, 18385 Gyeonggi-do (KR)
(74) Representative: Schweitzer, Klaus

(57) **Abstract**

The present invention relates to an indoor air sterilization and purification apparatus, which may perform both a space sterilization function for sterilizing and purifying microbial particles existing in the air of an indoor space and a surface sterilization function for sterilizing and disinfecting an object surface in an indoor space, thus to improve sterilization performance for the indoor space, and may proximately irradiate the object with sterilization light or spray a disinfectant without any special obstacle in movement, even if shapes and arrangement states of the objects existing in the indoor space are complicated, thus to improve the sterilization function.

## Description

### [RELATED APPLICATIONS]

This application claims priority to Korean Patent Application No. 10-2020-0137297, filed on October 22, 2020 in the Korean Intellectual Property Office, the entire disclosure of which is incorporated herein by reference.

### [BACKGROUND OF THE INVENTION]

### 1. Field of the Invention

The present invention relates to an indoor air sterilization and purification apparatus using a UV-C LED.

### 2. Description of the Related Art

Since 2000, serious infectious diseases such as severe acute respiratory syndrome (SARS), Influenza A virus subtype (H1N1), Middle East respiratory syndrome (MERS), and the recent new coronavirus disease 19 (COVID-19) have occurred and spread around the world, and there are very many infections and deaths, such that it is becoming a social issue periodically. Since the incidence frequency of each infectious disease is getting shorter, interest in prevention of the infectious diseases is increasing day by day. As targets to be applied the related law, the Infectious Disease Control and Prevention Act, are expanding, and disinfection standards are strengthening, certification evaluation standards of medical institutions and nursing institutions which are most vulnerable to the infectious diseases are also strengthening.

These viral infectious diseases may be largely divided into droplet infection, airborne infection, contact infection and the like. The droplet infection means that a body fluid of the infected person spatters through coughing, etc., and enters the respiratory tracts of other persons to cause infection. According to data of the Korean Association for Particle and Aerosol Research, a survival time of microorganisms in droplets varies depending on the type of microorganisms, and it is estimated that the variant virus of coronavirus can survive up to 24 hours. The airborne infection occurs when virus particles called droplet nuclei (with a size of 5 µm or less) float in the air and are inhaled by humans. The contact infection is infected through the skin or mucous membranes due to direct contacting with a patient, carrier, clothing, articles, and the like, to which pathogens are attached.

Therefore, in order to prevent the viral infectious diseases, a sterilization and disinfection apparatus capable of simultaneously performing sterilization and purification on air infectious sources as well as disinfection of spaces and object surfaces may be effective so as to cope with most infections through various infection routes.

Recently, a variety of air purifiers and sterilization and disinfection apparatuses for indoor spaces have been developed. However, the conventional indoor air sterilization and purification apparatuses are generally developed to an extent of adding a configuration that exhibits a sterilization function to an air purifier, or an extent of disposing a germicidal lamp that irradiates indoor air with sterilization light such as ultraviolet (UV) rays in an indoor space to sterilize and disinfect the indoor air, therefore they remain in a very simple form.

In the case of such a simple air sterilization purifier, there are problems that, in addition to the function of purifying the air by removing fine dusts, an efficiency of sterilization is not relatively high, and microorganisms existing in the air are filtered through a membrane filter that filters out the dusts, such that the infectious sources exist without losing infectious power with no special treatment subjected thereon. In addition, there are problems that a typical indoor air sterilization and purification apparatus exhibits relatively high efficiency of sterilization function in a region near the germicidal lamp, with which the sterilization light is irradiated, but the efficiency of the sterilization function is rapidly decreased as a distance from the germicidal lamp is increased, and thereby overall sterilization effects for the indoor space are very insignificant.

In order to solve these problems, a technique for a sterilization apparatus having an autonomous traveling function is being actively developed. Chinese Utility Model No. CN 209529745 U discloses a technique for a sterilization apparatus having an autonomous traveling function, but there are problems that frequent replacement is required due to a short lifespan of a fluorescent lamp, and environmental pollution occurs due to mercury generated during the replacement and disposal of the fluorescent lamps, and it is not possible to solve air pollution due to ozone generated by UV rays, as well as there are disadvantages in that it is not possible to determine whether sufficient sterilization has been achieved due to the lack of a technique for monitoring the microbial sterilization status.

In addition, Korean Patent Registration No. 10-2093161 discloses a technique for a smart automatic disinfection apparatus having an autonomous traveling function, which determines whether a region is contaminated by a first spectrum and performs sterilization on the region by irradiating UV rays to sterilize pollutants, and then determines whether an additional sterilization is required by acquiring a second spectrum of the reflected light, but there are disadvantages in that a sterilization range is limited only to sides of an object due to the structure of the light source, such that it is difficult to sterilize upper and lower surfaces of the object, and it is not possible to determine whether there is pollution in the air, because it is a technique that uses reflected light.

### [Prior Art Document]

### [Patent Document]

(Patent Document 1) Korean Patent Registration No. 10-2093161
(Patent Document 2) Chinese Utility Model CN 209529745 U

### [SUMMARY OF THE INVENTION]

In consideration of the above-mentioned circumstances, it is an object of the present invention to provide an indoor air sterilization and purification apparatus which may perform both a space sterilization function for sterilizing and purifying microbial particles existing in the air of an indoor space and a surface sterilization function for sterilizing and disinfecting an object surface in an indoor space, thus to improve sterilization performance for the indoor space.

In addition, another object of the present invention is to provide an indoor air sterilization and purification apparatus which is capable of moving a main germicidal lamp close to an object surface to irradiate the sterilization light, or spraying a disinfectant through a disinfectant spray module during a surface sterilization operation for sterilizing the object surface, thus to enhance a sterilization ability for the object, as well as capable of proximately irradiating the object with sterilization light or spraying the disinfectant without any special obstacle in movement, even if shapes and arrangement states of the objects existing in the indoor space are complicated, thus to improve the sterilization function.

Further, another object of the present invention is to provide an indoor air sterilization and purification apparatus which is capable of increasing an exposure time of sterilization light to microbial particles passing through an air purification case in a process of sterilizing and purifying air in an indoor space, thus to further improve air sterilization performance.

According to an aspect of the present invention, there is provided an indoor air sterilization and purification apparatus including: a driving unit which includes a plurality of wheels provided at a lower portion thereof to automatically travel in an indoor space; a circulation purification module mounted on the driving unit and configured to suck in an indoor air to filter, sterilize, or purify dusts or microbial particles contained in the indoor air and discharge the same; a sterilization and disinfection module which is mounted on the driving unit, includes a plurality of main germicidal lamps that emit sterilization light, wherein the main germicidal lamps are formed in a manner of adjusting arrangement positions thereof so that the main germicidal lamps are located close to an object to be disinfected, and is configured to irradiate a sterilization region between the object and the main germicidal lamps with UV-C; distance detection sensors mounted on the driving unit and the sterilization and disinfection module to detect a distance to an object existing in the indoor space therefrom; and a controller configured to control operations of the driving unit and the sterilization and disinfection module based on detection results of the distance detection sensors, wherein the main germicidal lamp is a UV-C LED or a UV-C lamp having a wavelength of 200 to 280 nm.

In one embodiment of the present invention, the main germicidal lamp may selectively emit only peaks including a wavelength of 275 nm.

A UV sterilization module of the present invention ensures a sterilization ability for a distant space. By applying an optical lens to an emission part of a light source, light is condensed to enable sterilization of the distant space. As the optical lens, a moth eye lens may be used, and one or more moth eye lenses may be installed on each LED. Preferably, one moth eye lens is installed thereon, and when UV rays are simultaneously emitted from adjacent LED light sources, an action in which lights from the moth eye lenses are mutually condensed occurs. When the optical lens is not installed, an effective distance (Wd) is about 30 mm, but when the optical lens is applied, the effective distance is increased more than 10 times, and thereby maximizing a disinfection ability at a distance. This may be applied to a Far UV-C sterilization apparatus that maximizes an optical power of a desired region using the UV sterilization module of the present invention.

In one embodiment of the present invention, the circulation purification module may be disposed on an upper portion of the driving unit, and the sterilization and disinfection module may be disposed movably up and down in a form surrounding an upper portion and a side space of the circulation purification module.

In one embodiment of the present invention, the circulation purification module may include: an air purification case mounted on the upper portion of the driving unit and having air inlets and an air outlet formed in one end portion and the other end portion thereof; an air filter mounted inside the air purification case to filter the air sucked through the air inlets; air germicidal lamps configured to emit sterilization light into an inner space of the air purification case to sterilize microbial particles contained in the air sucked through the air inlets; and a blowing fan operated to form an air flow so that air is sucked and discharged through the air inlets and the air outlet, wherein the air germicidal lamp may be a UV-C LED or a UV-C lamp having a wavelength of 200 to 280 nm.

Conventionally, small home appliances in which UV-C LEDs are inserted in a form of chips are widely sold on the market, but due to a high heat generation of the UV-C LED chips, these appliances cannot be used in a large-sized product for surface and space sterilization. In the present invention, by solving the high heat generation of the UV-C LED chips, these appliances are mounted on the large-sized product for surface and space sterilization to ensure a high sterilization ability.

In one embodiment of the present invention, the air filter may be formed in a cylindrical shape so as to allow air to pass therethrough from sides to a center direction, a plurality of air germicidal lamps may be mounted, and at least one lamp is arranged in a form of being inserted into a central portion of the air filter.

In one embodiment of the present invention, the plurality of air germicidal lamps are formed in a form in which a plurality of UV-C lamps or UV LEDs are mounted on a plate-shaped lamp substrate and mounted inside the air purification case, respectively, and at least two lamps of the plurality of air germicidal lamps may be disposed above the air filter to be vertically spaced apart from each other, and may be formed to guide the air flow passing through the air filter in a zigzag direction.

In one embodiment of the present invention, the sterilization and disinfection module includes: a sterilization base body disposed above the air purification case; a main driving unit configured to move the sterilization base body up and down; and a plurality of main germicidal lamps which are arranged along an outer circumference of the sterilization base body, move up and down along with the sterilization base body, extend longitudinally in a vertical direction, and have light emitting surfaces formed on outer surfaces thereof to emit sterilization light to an outer direction, wherein the main driving unit and the main germicidal lamp may be operated by a control of the controller.

In one embodiment of the present invention, upper ends of the plurality of main germicidal lamps are pivotably coupled to the sterilization base body about a horizontal rotation shaft, and pivot driving units are mounted on the sterilization base body, which independently pivot and move the plurality of main germicidal lamps about the horizontal rotation shaft, and are operated by a control of the controller.

In one embodiment of the present invention, the main driving unit may rotate and move the sterilization base body about a vertical rotation shaft provided at a central portion thereof.

In one embodiment of the present invention, the main germicidal lamp may have light emitting surfaces formed on an outer surface and an inner surface thereof, respectively, so as to emit the sterilization light in the outer direction and an inner center direction, respectively, with being arranged longitudinally in the vertical direction.

In one embodiment of the present invention, the main germicidal lamp may be made of a material that allows light to transmit through all the outer circumferential surfaces of the lamp with being arranged longitudinally in the vertical direction, so as to emit the sterilization light at 360° in all directions.

In one embodiment of the present invention, the controller may control operations of the modules so as to be operated simultaneously in both or selectively in any one of a space sterilization mode that operates the circulation purification module, and a surface sterilization mode that operates the sterilization and disinfection module.

In one embodiment of the present invention, the controller may control operations of the modules to continuously operate the circulation purification module so as to continuously perform the space sterilization mode in the indoor space, and operate the main germicidal lamp so as to perform the surface sterilization mode in which the surface of the object existing in the indoor space is irradiated with the sterilization light while moving an automatic traveling mobile body with being operated in the space sterilization mode.

In one embodiment of the present invention, the controller may control operations of the inventive apparatus: so as to continuously perform the space sterilization mode that operates the circulation purification module; and to perform the surface sterilization mode in which the surface of the object existing in the indoor space is irradiated with the sterilization light, by operating the main germicidal lamp while moving the driving unit with being operated in the space sterilization mode, as well as in the process of the surface sterilization mode on a lower surface of the object existing in the indoor space, the controller may control operations of the inventive apparatus so that the lower surface of the object is irradiated with the sterilization light, by moving the sterilization base body upward so as to be spaced apart from the lower surface of the object by a reference distance, and horizontally pivoting and moving the main germicidal lamp.

In one embodiment of the present invention, in the process of the surface sterilization mode on the lower surface of the object existing in the indoor space, the controller may control operations of the inventive apparatus so that the sterilization base body is rotated about the vertical rotation shaft by the main driving unit in a state of being irradiated with sterilization light by horizontally pivoting and moving the main germicidal lamp.

In one embodiment of the present invention, in the process of the surface sterilization mode on the upper surface of the object existing in the indoor space, the controller may control operations of the inventive apparatus so that the upper surface of the object is irradiated with the sterilization light emitted from the main germicidal lamp by moving the sterilization base body upward so as to be spaced apart from the upper surface of the object by a temporary set distance, and in this state, horizontally pivoting and moving the main germicidal lamp, and then moving the sterilization base body downward so as to be spaced apart from the upper surface of the object by a reference distance shorter than the temporary set distance.

In one embodiment of the present invention, the circulation purification module may further include a disinfectant spray module provided at a rear end of the air outlet.

In one embodiment of the present invention, the sterilization and disinfection module may further include a disinfectant spray module provided at an upper portion of the sterilization base body, or further include a disinfectant spray module at one end of the main germicidal lamp. In the case of an indoor surface or space sterilization apparatus using UV-C LED, a blind spot may occur where the LED light does not reach, and it is difficult to perform sterilization through killing of microorganisms in the area, such that it is supplemented to prevent the blind spot from occurring through mounting a module in a form of atomizing or steam spraying the disinfectant.

In one embodiment of the present invention, the disinfectant spray module may spray the disinfectant in an atomization or steam spray method depending on the type thereof. If the spray particle size is large, it is possible to create an environment in which microorganisms can proliferate, and thus the atomization or steam spray method can be adopted in a form of a spray in which liquid droplets are not formed.

The disinfectant of the present invention uses a spraying agent which is safe for the human body because no chemical toxicity or side effects are reported, and the disinfectant may be any one or more selected from the group consisting of sodium hypochlorite, ethanol, amphoteric surfactant, chlorhexidine, hydrogen peroxide, and benzalkonium chloride.

According to the present invention, it is possible to perform both the space sterilization function for sterilizing and purifying microbial particles existing in the air of the indoor space and the surface sterilization function for sterilizing and disinfecting an object surface in an indoor space, thus to improve sterilization performance for the indoor space.

In addition, it is possible to move the main germicidal lamp close to the object surface to emit the sterilization light, or spray a disinfectant through the disinfectant spray module during the surface sterilization operation for sterilizing the object surface, thus to enhance a sterilization ability for the object, as well as proximately irradiate the object with sterilization light or spray the disinfectant without any special obstacle in movement, even if shapes and arrangement states of the objects existing in the indoor space are complicated, thus to improve the sterilization function.

Further, it is possible to increase an exposure time of sterilization light to microbial particles passing through the air purification case in the process of sterilizing and purifying air in an indoor space, thus to further improve air sterilization performance.

### [BRIEF DESCRIPTION OF THE DRAWINGS]

The above and other objects, features and other advantages of the present invention will be more clearly understood from the following detailed description taken in conjunction with the accompanying drawings, in which:
FIG. 1 is a perspective view schematically illustrating an appearance of an indoor air sterilization and purification apparatus according to an embodiment of the present invention;
FIG. 2 is a view conceptually illustrating a configuration of the indoor air sterilization and purification apparatus according to the embodiment of the present invention;
FIG. 3 is a view illustrating an automatic movement state of the indoor air sterilization and purification apparatus according to the embodiment of the present invention;
FIG. 4 is a view conceptually illustrating an internal structure of a circulation purification module according to an embodiment of the present invention;
FIG. 5 is a view conceptually illustrating an operation structure of a sterilization and disinfection module according to an embodiment of the present invention;
FIG. 6 is a view schematically illustrating a state of a sterilization and disinfection operation on a lower surface of an object by the sterilization and disinfection module according to the embodiment of the present invention; and
FIG. 7 is a view schematically illustrating a state of the sterilization and disinfection operation on an upper surface of the object by the sterilization and disinfection module according to the embodiment of the present invention.

### [DETAILED DESCRIPTION OF THE INVENTION]

Hereinafter, preferred embodiments of the present invention will be described in detail with reference to the accompanying drawings. First, in denoting reference numerals to constitutional elements of respective drawings, it should be noted that the same elements will be denoted by the same reference numerals as much as possible even if they are illustrated in different drawings.

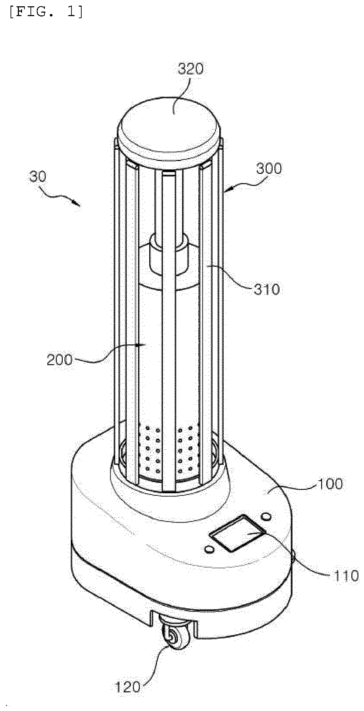

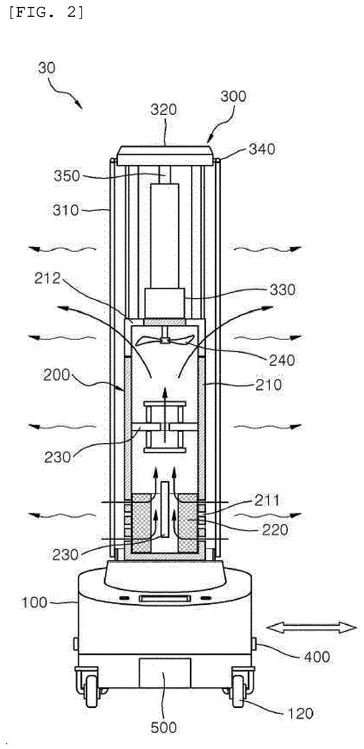

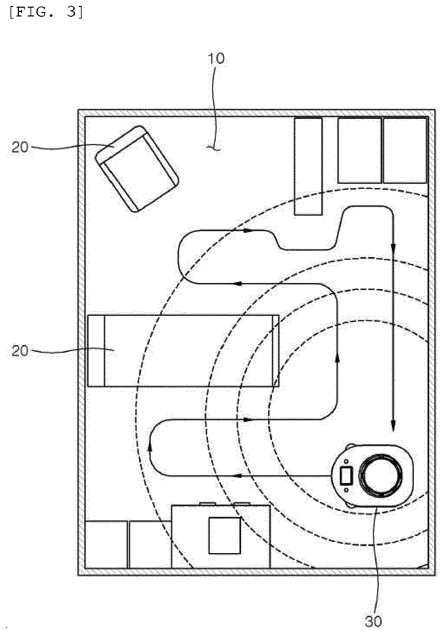

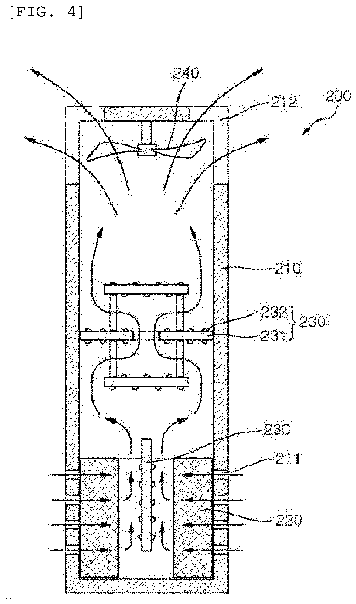

FIG. 1 is a perspective view schematically illustrating an appearance of an indoor air sterilization and purification apparatus according to an embodiment of the present invention, FIG. 2 is a view conceptually illustrating a configuration of the indoor air sterilization and purification apparatus according to the embodiment of the present invention, FIG. 3 is a view illustrating an automatic movement state of the indoor air sterilization and purification apparatus according to the embodiment of the present invention, and FIG. 4 is a view conceptually illustrating an internal structure of a circulation purification module according to an embodiment of the present invention.

An indoor air sterilization and purification apparatus according to an embodiment of the present invention is an apparatus capable of simultaneously performing a space sterilization function, which is an air purification function for an indoor space, and a surface sterilization function, which is a sterilization and disinfection function for surfaces of objects in the indoor space, and includes a driving unit 100 (hereinafter, also referred to as an automatic traveling mobile body 100), a circulation purification module 200, a sterilization and disinfection module 300, a distance detection sensor 400, and a controller 500.

The driving unit 100 is a mobile body capable of automatically traveling in an indoor space, and is configured to detect an obstacle by itself and automatically travel without a driving operation by a user. The driving unit may include wheels 120 for movement mounted at a lower portion thereof, buttons for operation and manipulation by the user, and an operation display unit 110 for displaying images, which are mounted on one side thereof.

In addition, the driving unit 100 may be equipped with various parts for automatic traveling, such as a driving unit (not illustrated) to rotate the wheels 120, and in particular, a plurality of distance detection sensors (400) to detect a distance to an object 20 existing in the indoor space therefrom. The controller 500 may calculate a moving route of the automatic traveling mobile body 100 based on detection results of the distance detection sensors 400, and control the driving unit 100 to move without colliding with an obstacle such as the object 20 in the indoor space 10.

The driving unit 100 is widely used in various industrial fields, and therefore will not be described in detail.

The circulation purification module 200 is mounted on the driving unit 100 to move along with the automatic traveling mobile body 100, and is configured to sterilize and purify fine particles and microbial particles contained in an indoor air by sucking in the indoor air.

The sterilization and disinfection module 300 is mounted on the driving unit 100 to move along with the automatic traveling mobile body 100, and includes a plurality of main germicidal lamps 310 that emit sterilization light. At this time, the main germicidal lamps 310 are formed in a manner of adjusting arrangement positions thereof so that the main germicidal lamps 310 are located close to the object 20 to be disinfected. The distance detection sensor 400 may also be mounted on the sterilization and disinfection module 300, and the controller 500 may adjust a proximal moving distance of the main germicidal lamps 310 to the object 20 to be disinfected based on the detection results of the distance detection sensors 400.

Meanwhile, the controller 500 may control operations of the modules so as to be operated simultaneously in both or selectively in any one of a space sterilization mode that operates the circulation purification module 200 to purify the air in the indoor space 10, and a surface sterilization mode that operates the sterilization and disinfection module 300 to perform sterilization and disinfection on the surface of the object 20.

According to the above-described configuration, the indoor air sterilization and purification apparatus 30 according to the embodiment of the present invention may simultaneously perform the space sterilization mode through the circulation purification module 200 and the surface sterilization mode through the sterilization and disinfection module 300 to simultaneously perform sterilization and disinfection on the object in the indoor space 10.

More specifically, the controller may continuously operate the circulation purification module 200 so as to continuously perform the space sterilization mode in the indoor space, and operate the main germicidal lamp 310 so as to perform the surface sterilization mode in which the surface of the object 20 existing in the indoor space 10 is irradiated with the sterilization light while moving an automatic traveling mobile body 100 with being operated in the space sterilization mode. At this time, in the surface sterilization mode, by moving the main germicidal lamp 310 close to the surface of the object 20 to be disinfected, the surface of the object 20 to be disinfected is proximately irradiated with the sterilization light, thereby effects of sterilizing and disinfecting the object surface may be improved.

For example, as shown in FIG. 3, when a plurality of objects 20 exist in the indoor space 10, the indoor air sterilization and purification apparatus 30 according to the embodiment of the present invention detects the object 20 using the distance detection sensor 400 etc., and the controller 500 calculates the moving route of the driving unit 100 based on the detection results. Therefore, as shown by arrows in FIG. 3, it is possible to move the entire region of the indoor space 10 so as to be located close to the object 20 along the moving route.

In this way, the indoor air sterilization and purification apparatus 30 moves the entire region of the indoor space 10, such that all objects 20 of the indoor space 10 are proximately irradiated with the sterilization light from the main germicidal lamp 310, and thereby the sterilization and disinfection function on the surface of the object 20 may be perfectly performed in full.

Meanwhile, as shown in FIGS. 1 and 2, the circulation purification module 200 is disposed on an upper portion of the driving unit 100, and the sterilization and disinfection module 300 may be disposed movably up and down in a form surrounding an upper portion and a side space of the circulation purification module 200.

First, a configuration of the circulation purification module 200 according to the embodiment of the present invention will be described in more detail.

As shown in FIGS. 2 and 4, the circulation purification module 200 may include: an air purification case 210 mounted on an upper portion of the automatic traveling mobile body 100 and having air inlets 211 and an air outlet 212 formed on one side and the other side thereof; an air filter 220 mounted inside the air purification case 210 to filter the air sucked through the air inlets 211; air germicidal lamps 230 configured to emit sterilization light into an inner space of the air purification case 210 to sterilize microbial particles contained in the air sucked through the air inlets 211; and a blowing fan 240 operated to form an air flow so that air is sucked and discharged through the air inlets 211 and the air outlet 212. Herein, the blowing fan 240 and the air germicidal lamp 230 are operated by a control of the controller 500.

The air inlets 211 are formed in sides of a lower end portion of the air purification case 210, and the air outlet 212 is formed in an upper end portion of the air purification case 210, such that external air may be introduced into the inner space through the sides of the lower end portion of the air purification case 210 to be filtered, and then discharged to an outside through the upper end portion.

In this case, the air filter 220 may be applied as a filter capable of filtering both fine dusts and microbial particles, and may be formed in a cylindrical shape to allow air to pass therethrough from the sides to a center direction.

A plurality of air germicidal lamps 230 may be provided so as to sterilize both microbial particles filtered by the air filter 220 and microbial particles which are not filtered by the air filter 220 inside the air purification case 210. Herein, at least one lamp may be disposed to emit sterilization light toward the air filter 220 in a form of being inserted into a central portion of the air filter 220. The remaining lamps of the plurality of air germicidal lamps 230 may be disposed above the air filter 220 to sterilize the microbial particles passing through the air filter 220.

As shown in FIG. 4, these air germicidal lamps 230 may be formed in a form in which a plurality of UV LEDs 232 capable of emitting UV rays are mounted on a plate-shaped lamp substrate 231, and the plurality of air germicidal lamps 230 disposed above the air filter 220 may be formed in such a way that they are disposed above the air filter 220 to be vertically spaced apart from each other, and may be formed to guide the air flow passing through the air filter 220 in a zigzag direction.

To this end, the plurality of air germicidal lamps 230 may be disposed to be shifted from each other in a vertical direction inside the air purification case 210.

According to this structure, the microbial particles filtered by the air filter 220 are sterilized by the air sterilizing lamp 230 inserted in the central portion of the air filter 220, and the microbial particles passing through the air filter 220 flow in an upward zigzag direction by the plurality of air sterilizing lamps 230 as shown in FIG. 4, such that the time at which the microbial particles stay inside the air purification case 210 is increased. Therefore, an exposure time at which the microbial particles are irradiated with the sterilization light emitted from the air germicidal lamp 230 is increased, and thereby the sterilization function is improved.

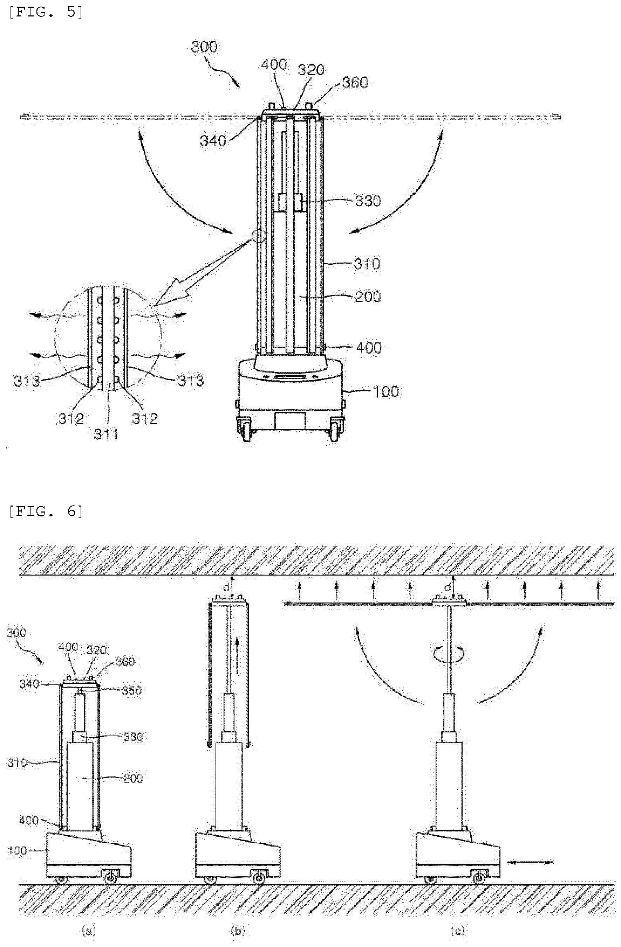

Next, the configuration of the sterilization and disinfection module 300 according to an embodiment of the present invention will be described in more detail with reference to FIGS. 5 to 7.

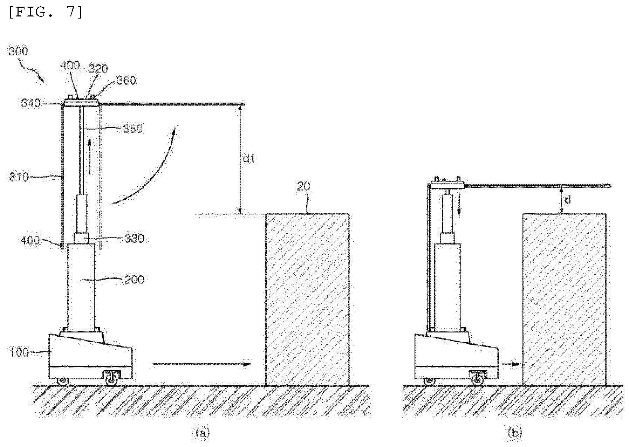

FIG. 5 is a view conceptually illustrating an operation structure of the sterilization and disinfection module according to an embodiment of the present invention, FIG. 6 is a view schematically illustrating a state of a sterilization and disinfection operation on a lower surface of an object by the sterilization and disinfection module according to the embodiment of the present invention, and FIG. 7 is a view schematically illustrating a state of the sterilization and disinfection operation on an upper surface of the object by the sterilization and disinfection module according to the embodiment of the present invention.

The sterilization and disinfection module 300 according to an embodiment of the present invention includes: a sterilization base body 320 disposed above the air purification case 210; a main driving unit 330 configured to move the sterilization base body 320 up and down; and a plurality of main germicidal lamps 310 which are arranged along an outer circumference of the sterilization base body 320, move up and down along with the sterilization base body 320, extend longitudinally in a vertical direction, and have light emitting surfaces formed on outer surfaces thereof to emit sterilization light to an outer direction. Herein, the main driving unit 330 and the main germicidal lamp 310 are operated by a control of the controller 500.

Upper ends of the plurality of main germicidal lamps 310 are pivotably coupled to the sterilization base body 320 about a horizontal rotation shaft 340, and pivot driving units 360 are mounted on the sterilization base body 320, which independently pivot and move the plurality of main germicidal lamps 310 about the horizontal rotation shaft 340 to be operated by a control of the controller 500.

In addition, the main driving unit 330 may be configured to move the sterilization base body 320 up and down, as well as to rotate and move the sterilization base body 320 about the vertical rotation shaft 350 provided at the central portion thereof.

In this case, the main germicidal lamp 310 may have light emitting surfaces formed on an outer surface and an inner surface thereof, respectively, so as to emit the sterilization light in the outer direction and an inner center direction, respectively, with being arranged longitudinally in the vertical direction. For example, as shown in an enlarged view of FIG. 5, it may be formed in a form in which a lamp substrate 311 formed longitudinally in the vertical direction is disposed at the central portion, a plurality of UV LEDs 312 capable of emitting UV rays are mounted on both sides of the lamp substrate 311, respectively, and transparent protective covers 313 are mounted at the outside.

According to the above-described configuration, a plurality of main germicidal lamps 310 are arranged to be spaced apart from each other in a circumferential direction along the outer circumferential surface of the sterilization base body 320, and each of the main germicidal lamps 310 can emit sterilization light toward the inner center direction and the outer direction.

Since the main germicidal lamp 310 can emit the sterilization light not only in the outer direction but also in the inner center direction, it may be configured to perform the air sterilization function of the circulation purification module 200 disposed in the inner center direction of the main germicidal lamp 310.

The main germicidal lamp 310 may be made of a material that allows light to transmit through all the outer circumferential surfaces of the lamp, so as to emit the sterilization light at 360° in all directions.

For example, the air purification case 210 of the circulation purification module 200 may be made of a transparent material, and the air germicidal lamp 230 that emits sterilization light into the interior space of the air purification case 210 is not separately provided, so as to emit the sterilization light into the interior space of the air purification case 210 by the main germicidal lamp 310 of the sterilization and disinfection module 300. In this case, the sterilization light in the outer direction of the main germicidal lamp 310 performs the sterilization and disinfection function on the surface of the object, and the sterilization light in the inner direction performs the sterilization and disinfection function on the microbial particles passing through the air purification case 210.

Even if it is configured that the sterilization light of the main germicidal lamp 310 is emitted in both the outer and inner directions as described above, of course the above-described air germicidal lamp 230 may be separately mounted inside the air purification case 210.

The indoor air sterilization and purification apparatus 30 according to the embodiment of the present invention may simultaneously perform the space sterilization mode and the surface sterilization mode as described above. In a process of the surface sterilization mode on the surface of the object 20, it is configured that the surface of the object 20 is irradiated with the sterilization light emitted from the main germicidal lamp 310 at a position close thereto.

For example, in the process of the surface sterilization mode on the lower surface of the object 20 to be disinfected existing in the indoor space, the controller 500 may control operations of the inventive apparatus so that, as shown in FIG. 6 (a) and (b), the lower surface of the object 20 is irradiated with the sterilization light, by moving the sterilization base body 320 upward so as to be spaced apart from the lower surface of the object 20 by a reference distance d, and in this state, as shown in FIG. 6 (c), the lower surface of the object 20 is irradiated with the sterilization light by horizontally pivoting and moving the main germicidal lamp 310 about the horizontal rotation shaft 340 by a pivot driving unit 360. In this case, the irradiation may be variously adjusted depending on a spatial arrangement state of the object 20 to be disinfected, such as all the plurality of main germicidal lamps 310 may be horizontally pivoted, and only some of the lamps may be selectively horizontally pivoted.

In addition, in this state, the sterilization base body 320 may be operated by a control of the controller 500 to be rotated about the vertical rotation shaft 350 by the main driving unit 330.

Depending on the operating state of the sterilization and disinfection module 300, since the lower surface of the object 20 may be irradiated with the sterilization light in close proximity by a reference distance d, the sterilization and disinfection function on the lower surface of the object 20 may be improved. In addition, since the main germicidal lamp 310 is rotated about the vertical rotation shaft 350, a relatively large region may be simultaneously irradiated with the sterilization light, and thereby decreasing the sterilization time.

Meanwhile, in the process of the surface sterilization mode on the upper surface of the object 20 to be disinfected existing in the indoor space, as shown in FIG. 7 (a), the controller 500 may control operations of the inventive apparatus so that the upper surface of the object 20 is proximately irradiated with the sterilization light emitted from the main germicidal lamp 310 by moving the sterilization base body 320 upward so as to be spaced apart from the upper surface of the object 20 by a temporary set distance dl, and in this state, horizontally pivoting and moving the main germicidal lamp 310, and then, as shown in FIG. 7 (b), moving the sterilization base body 320 downward so as to be spaced apart from the upper surface of the object 20 by a reference distance d shorter than the temporary set distance dl.

At this time, all the plurality of main germicidal lamps 310 may be horizontally pivoted and moved, but as shown in FIG. 7, only some of the lamps may be moved close to the upper surface of the object 20 with being horizontally pivoted and moved, and in this state, the inventive apparatus may be controlled so that the entire region of the upper surface of the object 20 is irradiated with the sterilization light by moving the driving unit 100.

By such various control methods, even if the shape and arrangement state of the object 20 in the indoor space is complicated, the upper and lower surfaces of the object 20 may be proximately irradiated with the sterilization light without any special obstacle on the moving route, thereby improving the sterilization function.

The above description is merely illustrative of the technical idea of the present invention, and those skilled art to which the present invention pertains will appreciate that various modifications and variations are possible without departing from the essential characteristics of the present invention. Therefore, the embodiments disclosed in the present invention are intended to describe the technical spirit of the present invention, and are not intended to limit the same, as well as the scope of the technical spirit of the present invention is not limited to these embodiments. It should be understood that the protective scope of the present invention is interpreted by the claims below, and all technical ideas within the equivalent range are included in the scope of the present invention.

### [Description of Reference Numerals]

100: Driving unit
200: Circulation purification module
210: Air purification case
211: Air inlet
212: Air outlet
220: Air filter
230: Air germicidal lamp
240: Blowing fan
300: Sterilization and disinfection module
310: Main germicidal lamp
320: Sterilization base body
330: Main driving unit
340: Horizontal rotation shaft
350: Vertical rotation shaft
360: Rotation driving unit
370: Disinfectant spray module
400: Distance detection sensor
500: Controller

## Claims

1. An indoor air sterilization and purification apparatus comprising:
a driving unit which includes a plurality of wheels provided at a lower portion thereof to automatically travel in an indoor space;
a sterilization and disinfection module which is mounted on the driving unit, includes a plurality of main germicidal lamps that emit sterilization light, wherein the main germicidal lamps are formed in a manner of adjusting arrangement positions thereof so that the main germicidal lamps are located close to an object to be disinfected, and is configured to irradiate a sterilization region between the object and the main germicidal lamps with UV-C;
a circulation purification module mounted on the driving unit and configured to suck in an indoor air to filter, sterilize, or purify dusts or microbial particles contained in the indoor air and discharge the same;
distance detection sensors mounted on the driving unit and the sterilization and disinfection module to detect a distance to an object existing in the indoor space therefrom; and
a controller configured to control operations of the driving unit and the sterilization and disinfection module based on detection results of the distance detection sensors,
wherein the main germicidal lamp is a UV-C LED or a UV-C lamp having a wavelength of 200 to 280 nm.

2. The indoor air sterilization and purification apparatus according to claim 1, wherein the circulation purification module is disposed on an upper portion of the driving unit, and
the sterilization and disinfection module is disposed movably up and down in a form surrounding an upper portion and a side space of the circulation purification module.

3. The indoor air sterilization and purification apparatus according to claim 2, wherein the circulation purification module comprises:
an air purification case mounted on the upper portion of the driving unit and having air inlets and an air outlet formed in one end portion and the other end portion thereof;
an air filter mounted inside the air purification case to filter the air sucked through the air inlets;
air germicidal lamps configured to emit sterilization light into an inner space of the air purification case to sterilize microbial particles contained in the air sucked through the air inlets; and
a blowing fan operated to form an air flow so that air is sucked and discharged through the air inlets and the air outlet; and
a disinfectant spray module provided at a rear end of the air outlet.

4. The indoor air sterilization and purification apparatus according to claim 3, wherein the air filter is formed in a cylindrical shape so as to allow air to pass therethrough from sides to a center direction,
a plurality of air germicidal lamps are mounted thereon, and at least one lamp is arranged in a form of being inserted into a central portion of the air filter, and
the air germicidal lamp is a UV-C LED system or a UV-C lamp having a wavelength of 200 to 280 nm.

5. The indoor air sterilization and purification apparatus according to claim 4, wherein the plurality of air germicidal lamps are formed in a form in which a plurality of UV-C lamps or UV LEDs are mounted on a plate-shaped lamp substrate and mounted inside the air purification case, respectively, and
at least two lamps of the plurality of air germicidal lamps may be disposed above the air filter to be vertically spaced apart from each other, and may be formed to guide the air flow passing through the air filter in a zigzag direction.

6. The indoor air sterilization and purification apparatus according to claim 2, wherein the sterilization and disinfection module comprises:
a sterilization base body disposed above the air purification case;
a main driving unit configured to move the sterilization base body up and down;
a plurality of main germicidal lamps which are arranged along an outer circumference of the sterilization base body, move up and down along with the sterilization base body, extend longitudinally in a vertical direction, and have light emitting surfaces formed on outer surfaces thereof to emit sterilization light to an outer direction; and
a disinfectant spray module provided at an upper portion of the sterilization base body, at an upper end of the UV-C LED system, or at one end of the main germicidal lamp,
wherein the main driving unit and the main germicidal lamp are operated by a control of the controller.

7. The indoor air sterilization and purification apparatus according to claim 6, wherein upper ends of the plurality of main germicidal lamps are pivotably coupled to the sterilization base body about a horizontal rotation shaft,
pivot driving units are mounted on the sterilization base body, which independently pivot and move the plurality of main germicidal lamps about the horizontal rotation shaft, and are operated by a control of the controller, and
the main driving unit is configured to rotate and move the sterilization base body about a vertical rotation shaft provided at a central portion thereof.

8. The indoor air sterilization and purification apparatus according to claim 7, wherein the main germicidal lamp is made of a material that allows light to transmit through all the outer circumferential surfaces of the lamp with being arranged longitudinally in the vertical direction, so as to emit the sterilization light at 360° in all directions.

9. The indoor air sterilization and purification apparatus according to any one of items 1 to 8, wherein the controller controls operations of the modules so as to be operated simultaneously in both or selectively in any one of a space sterilization mode that operates the circulation purification module, and a surface sterilization mode that operates the sterilization and disinfection module.

10. The indoor air sterilization and purification apparatus according to claim 9, wherein the controller controls operations of the apparatus so as to perform the surface sterilization mode in which the surface of the object existing in the indoor space is irradiated with the sterilization light, by operating the main germicidal lamp while moving the driving unit with being operated in the space sterilization mode;
in the process of the surface sterilization mode on a lower surface of the object existing in the indoor space,
the controller controls operations of the apparatus so that the lower surface of the object is irradiated with the sterilization light, by moving the sterilization base body upward so as to be spaced apart from the lower surface of the object by a reference distance, and horizontally pivoting and moving the main germicidal lamp; and
the controller controls operations of the apparatus so that the sterilization base body is rotated about the vertical rotation shaft by the main driving unit in a state of being irradiated with sterilization light by horizontally pivoting and moving the main germicidal lamp.

11. The indoor air sterilization and purification apparatus according to claim 9, wherein in the process of the surface sterilization mode on the upper surface of the object existing in the indoor space,
the controller controls operations of the apparatus so that the upper surface of the object is irradiated with the sterilization light emitted from the main germicidal lamp by moving the sterilization base body upward so as to be spaced apart from the upper surface of the object by a temporary set distance, and in this state, horizontally pivoting and moving the main germicidal lamp, and then moving the sterilization base body downward so as to be spaced apart from the upper surface of the object by a reference distance shorter than the temporary set distance.

12. The indoor air sterilization and purification apparatus according to claim 3 or 6, wherein the disinfectant spray module sprays the disinfectant in an atomization or steam spray method depending on the type thereof.

13. The indoor air sterilization and purification apparatus according to claim 1 or 3, wherein the main germicidal lamp or the air germicidal lamp selectively emits only peaks including a wavelength of 275 nm.

14. The indoor air sterilization and purification apparatus according to claim 1, wherein at least one moth eye lens is installed on at least one module of the UV-C LED system.
